(19) **Europäisches Patentamt / European Patent Office / Office européen des brevets**

(11) **EP 4 159 118 B1**

(12) **FASCICULE DE BREVET EUROPEEN**

(45) Date de publication et mention
de la délivrance du brevet:
**30.07.2025 Bulletin 2025/31**

(21) Numéro de dépôt: **22198522.9**

(22) Date de dépôt: **28.09.2022**

(51) Classification Internationale des Brevets (IPC):
*A61B 5/021* (2006.01)    *A61B 5/107* (2006.01)
*A61B 5/00* (2006.01)    *A61B 5/026* (2006.01)
*A61B 8/04* (2006.01)    *A61B 8/15* (2006.01)
*A61B 5/02* (2006.01)

(52) Classification Coopérative des Brevets (CPC):
**A61B 5/02116; A61B 5/02125; A61B 5/0261;
A61B 5/1075; A61B 5/681; A61B 8/04; A61B 8/15;**
A61B 5/02007; A61B 2560/0223; A61B 2562/0204;
A61B 2562/0233; A61B 2562/046; A61B 2562/066

(54) **PROCÉDÉ SANS BRASSARD DE DÉTERMINATION DE LA PRESSION ARTÉRIELLE D'UN UTILISATEUR**

MANSCHETTENLOSES VERFAHREN ZUR BESTIMMUNG DES BLUTDRUCKS EINES BENUTZERS

CUFF-LESS METHOD FOR DETERMINING A USER'S BLOOD PRESSURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.09.2021 FR 2110386**

(43) Date de publication de la demande:
**05.04.2023 Bulletin 2023/14**

(73) Titulaire: **Commissariat à l'Energie Atomique et aux Energies Alternatives
75015 Paris (FR)**

(72) Inventeurs:
• **BLANDIN, Pierre**
  **Grenoble (FR)**
• **BONNET, Stéphane**
  **Grenoble (FR)**
• **GERBELOT BARILLON, Rémi**
  **Grenoble (FR)**
• **CAILLAT, Patrice**
  **Grenoble (FR)**

(74) Mandataire: **INNOV-GROUP
209 Avenue Berthelot
69007 Lyon (FR)**

(56) Documents cités:
**US-A1- 2010 081 912    US-A1- 2018 078 155**

• **NABEEL P M ET AL: "Bi-Modal Arterial Compliance Probe for Calibration-Free Cuffless Blood Pressure Estimation", IEEE TRANSACTIONS ON BIOMEDICAL ENGINEERING, IEEE, USA, vol. 65, no. 11, 1 November 2018 (2018-11-01), pages 2392 - 2404, XP011691969, ISSN: 0018-9294, [retrieved on 20181017], DOI: 10.1109/TBME.2018.2866332**

## Description

## DOMAINE TECHNIQUE

Le domaine technique est la caractérisation de la pression artérielle sans compression.

## ART ANTERIEUR

La plupart des dispositifs permettant de caractériser la pression artérielle utilisent un capteur de pression couplé à un brassard de compression disposé sur un membre, généralement un bras. La caractérisation de la pression artérielle est effectuée en mesurant la pression exercée par le brassard en un ou plusieurs instants caractéristiques. Le capteur de pression ou le capteur acoustique sont sensibles aux battements cardiaques et à leur amplitude.

Les dispositifs utilisés par le personnel médical (méthode auscultatoire) sont composés d'un brassard dont la pression est contrôlée et lue par le médecin, associé généralement à un stéthoscope. Lorsque le brassard se dégonfle, on détecte l'apparition et la disparition de sons, appelés sons de Korotkoff. La pression appliquée par le brassard lors de l'apparition et la disparition des sons correspond respectivement à la pression systolique et à la pression diastolique.

Dans les tensiomètres grand public, un capteur de pression détermine la pression d'air dans le brassard. La compression du brassard est effectuée de manière à obtenir au préalable une occlusion artérielle. Lors de la déflation du brassard, des oscillations de pression apparaissent. Les oscillations augmentent jusqu'à atteindre, transitoirement, une amplitude maximale. A cet instant la pression dans le brassard est considérée égale à la pression artérielle moyenne. A partir de l'amplitude maximale détectée, les pressions artérielles systolique et diastolique sont estimées sur la base de lois empiriques.

Cependant, le recours à un dispositif comportant un brassard suppose des phases de compression régulières si l'on souhaite effectuer une mesure de la pression en continu. Cela constitue une source d'inconfort, liée à la fois à la perception de la compression et au bruit de la pompe activant la compression du brassard. En outre, une occlusion répétée selon une fréquence trop importante peut présenter un risque.

US2010081912 et US2018078155 décrivent des dispositifs pour mesurer la pression artérielle en combinant des modalités optiques et acoustiques.

Récemment, des développements ont été menés, de façon à pouvoir effectuer des mesures de la pression artérielle dites «cuffless» (sans brassard). La publication [1] Nabeel M. « Bi-modal arterial compliance probe for calibration-free cuffless blood pressure estimation", IEEE transactions on biomedical engineering, Vol. 5, N° 11, November 2018, décrit un dispositif couplant une modalité acoustique et une modalité optique pour estimer la pression artérielle d'un utilisateur, et cela sans recours à une compression d'un membre de l'utilisateur à l'aide d'un brassard. La modalité acoustique permet une mesure de l'évolution du diamètre de l'artère entre deux valeurs extrêmes, correspondant respectivement à la systole et la diastole. La modalité optique permet, selon les principes de la PPG (Photo-pléthysmographie infra-rouge), d'estimer une vitesse d'onde de pouls (VOP) entre deux points de mesure, distants l'un de l'autre, le long de l'artère. La vitesse d'onde de pouls est usuellement désignée par l'acronyme PWV (Pulse Wave Velocity). Les mesures résultant des deux modalités sont combinées de façon à pouvoir estimer la pression artérielle.

Les principes exposés dans la publication [1] peuvent être mis en œuvre pour concevoir un dispositif porté par un utilisateur, et permettant un suivi continu de la pression artérielle, en réduisant la gêne ressentie par l'utilisateur. Cependant les mesures effectuées dans les deux modalités peuvent être affectées par des incertitudes liées au positionnement des composants actifs (sources de lumière, capteurs acoustiques ou optiques), par rapport à l'artère. Ainsi, lorsque le dispositif est appliqué sur le corps d'un utilisateur, les capteurs doivent être correctement disposés précisément par rapport à l'artère de façon que la variation du diamètre et la vitesse d'onde de pouls soient correctement estimés.

Aussi, le dispositif précédemment décrit suppose un positionnement précis sur la peau de l'utilisateur. Une autre difficulté est liée aux mouvements de l'utilisateur, qui peuvent entraîner une variation de la position des capteurs par rapport à l'artère. L'invention décrite ci-après permet de surmonter ces difficultés.

## EXPOSE DE L'INVENTION

Un premier objet de l'invention est un dispositif d'estimation d'une pression artérielle d'un utilisateur, le dispositif étant destiné à être porté par l'utilisateur, le dispositif comportant :

- un support, destiné à être appliqué contre la peau de l'utilisateur ;
- plusieurs sources de lumière, disposées sur le support, configurées pour émettre une lumière vers la peau de l'utilisateur lorsqu'elles sont activées ;
- plusieurs photodétecteurs, disposés sur le support, distants de chaque source de lumière, configurés pour détecter une lumière émanant de la peau de l'utilisateur suite à une activation d'au moins une source de lumière, chaque photodétecteur formant, avec ladite source de lumière, un couple source-photodétecteur ;
- plusieurs transducteurs acoustiques, **chacun** comportant au moins :

  • un émetteur acoustique, configuré pour émettre une onde acoustique à travers la peau ;
  • et un détecteur acoustique, configuré pour détecter une onde acoustique réfléchie dans le corps de l'utilisateur, se propageant à travers la peau ;

- une unité de sélection acoustique, programmée pour :

  • prendre en compte un critère de sélection acoustique ;
  • sélectionner un émetteur acoustique et un détecteur acoustique parmi les transducteurs acoustique, la sélection étant effectuée en fonction d'un signal acoustique détecté par chaque détecteur acoustique suite à une émission d'une onde acoustique par au moins un émetteur acoustique;

- une unité de sélection optique, configurée pour :

  • prendre en compte un critère de sélection optique ;
  • sélectionner un premier couple source de lumière - photodétecteur, comportant une première source de lumière et un premier photodétecteur, choisis parmi les sources de lumière et les photodétecteurs, et un deuxième couple source de lumière - photodétecteur, comportant une deuxième source de lumière et un deuxième photodétecteur, choisis parmi les sources de lumière et les photodétecteurs, la sélection étant effectuée en fonction des signaux détectés par le premier photodétecteur et le deuxième photodétecteur suite à une activation de la première source de lumière et de la deuxième source de lumière ;

- une unité centrale, programmée pour :

  • • calculer un décalage temporel entre deux échos dans le signal détecté par le détecteur acoustique sélectionné;
  • calculer un décalage temporel entre des signaux détectés par le premier photodétecteur et le deuxième photodétecteur;
  • estimer une pression artérielle à partir des décalages temporels calculés.

L'unité centrale peut être programmée pour :

• estimer un diamètre artériel à partir du décalage temporel entre les échos du signal détecté par le détecteur acoustique sélectionné;
• estimer une vitesse d'onde de pouls, à partir du décalage temporel entre les signaux détectés par le premier photodétecteur et le deuxième photodétecteur ;
• estimer la pression artérielle, en fonction du diamètre artériel et de l'onde de pouls estimés.

De préférence, la source de lumière émet une lumière dans une bande spectrale comprise entre 500 nm et 1200 nm.

Le dispositif peut comporter :

- un premier groupe de sources de lumière et de photodétecteurs ;
- un deuxième groupe de sources de lumière et de photodétecteurs, distant du premier groupe de sources de lumière et de photodétecteurs.

L'unité de sélection optique est alors configurée pour :

• sélectionner la première source de lumière et le premier photodétecteur parmi les sources de lumière et les photodétecteurs du premier groupe ;
• sélectionner la deuxième source de lumière et le deuxième photodétecteur parmi les sources de lumière et les photodétecteurs du deuxième groupe.

Selon une possibilité, le critère acoustique étant un rapport signal sur bruit, l'unité de sélection acoustique est configurée pour :

- estimer un rapport signal sur bruit de chaque signal détecté par d'un détecteur acoustique ;
- sélectionner le détecteur acoustique pour lequel le rapport signal sur bruit le plus élevé.

Le critère acoustique peut être une intensité du signal détecté par un détecteur acoustique. l'unité de sélection acoustique est alors configurée pour

- estimer une intensité de chaque signal détecté par un détecteur acoustique ;
- sélectionner le détecteur acoustique dont l'intensité est la plus élevée.

Selon une possibilité, le critère optique étant un critère de corrélation, l'unité de sélection optique est configurée pour :

- estimer une corrélation temporelle entre les signaux détectés en différents instants par des photodétecteurs de chaque couple source de lumière-photodétecteur;
- sélectionner la première source de lumière et le premier photodétecteur ainsi que la deuxième source de lumière et le deuxième photodétecteur en fonction de la corrélation temporelle estimée.

Selon une possibilité, le critère optique est un critère d'amplitude, l'unité de sélection optique est configurée pour :

- estimer une amplitude d'une évolution temporelle de signaux détectés en plusieurs instants par des photodétecteurs de chaque couple source de lumière-photodétecteur;
- sélectionner la première source de lumière et le premier photodétecteur ainsi que la deuxième source de lumière et le deuxième photodétecteur en fonction de l'amplitude.

Selon une possibilité, le critère optique est un critère de forme. L'unité de sélection optique est configurée pour :

- prendre en compte une forme temporelle prédéterminée ;
- déterminer une évolution temporelle des signaux détectés, en différents instants par les photodétecteurs de chaque couple source de lumière-photodétecteur;
- sélectionner la première source de lumière et le premier photodétecteur ainsi que la deuxième source de lumière et le deuxième photodétecteur en fonction d'une corrélation entre l'évolution temporelle des signaux détectés et la forme temporelle prédéterminée.

Un autre objet de l'invention est un procédé d'estimation d'une pression artérielle à l'aide d'un dispositif selon le premier objet de l'invention, le procédé comportant :

a) disposition du support sur la peau d'un utilisateur, face à une artère;
b) émission d'au moins une onde acoustique incidente par un émetteur acoustique et acquisition de signaux acoustiques par un détecteur acoustique, chaque signal acoustique détecté comportant des échos représentatifs de réflexions de l'onde acoustique incidente par l'artère, l'étape b) étant réalisée pour différents émetteurs acoustique et/ou différents détecteurs acoustiques, de telle sorte que chaque signal acoustique détecté est associé à un émetteur acoustique et à un détecteur acoustique ;
c) à l'aide de l'unité de sélection acoustique :

- prise en compte d'un critère de sélection acoustique ;
- sélection d'un émetteur acoustique et d'un détecteur acoustique, en fonction d'une confrontation entre chaque signal acoustique détecté, lors de l'étape b), et le critère de sélection acoustique ;

d) pour chaque source de lumière, émission d'une lumière incidente vers la peau de l'utilisateur et détection d'un rayonnement rétrodiffusé par au moins un photodétecteur, chaque photodétecteur générant un signal optique représentatif de l'intensité du rayonnement rétrodiffusé ;
e) à l'aide de l'unité de sélection optique :

- prise en compte d'un critère de sélection optique ;
- sélection de deux couples source de lumière - photodétecteur, chaque couple comportant une source de lumière et un photodétecteur, en fonction d'une confrontation entre chaque signal optique issu de chaque photodétecteur et du critère de sélection optique ;

f) émission d'une onde acoustique incidente par le transducteur acoustique sélectionné lors de c), et formation d'un signal acoustique représentatif d'échos suite à des réflexions de l'onde acoustique incidente par l'artère ;
g) activation des sources de lumière de chaque couple source de lumière-photodétecteur sélectionné lors de e) et formation, par chaque photodétecteur de chaque couple, d'un signal optique représentatif de l'intensité du rayonnement rétrodiffusé par l'artère ;
h) en fonction d'un décalage temporel entre deux échos du signal acoustique, et d'un décalage temporel entre les signaux optiques formés par chaque photodétecteur en différents instants, estimation de la pression artérielle de l'utilisateur.

Selon une possibilité, l'étape h) comporte :

- h1) en fonction du signal acoustique formé, estimation du diamètre de l'artère;
- h2) en fonction des signaux optiques formés, en différents instants, par chaque photodétecteur sélectionné, estimation d'une vitesse d'onde de pouls ;
- h3) à partir du diamètre de l'artère résultant de la sous-étape h1) et de la vitesse d'onde de pouls résultant de la sous-étape h2), estimation d'une pression artérielle de l'utilisateur.

La sous-étape h2) peut comporter une estimation d'un décalage temporel entre les signaux optiques respectivement formés par le premier photodétecteur et le deuxième photodétecteur. Les étapes a) à e) peuvent constituent une phase de calibration du dispositif, les étapes f) à h) étant réitérées entre deux calibrations successives.

Selon une possibilité, le dispositif comporte :

- un premier groupe de sources de lumière et de photodétecteurs ;
- un deuxième groupe de sources de lumière et de photodétecteurs, distant du premier groupe de sources de lumière et de photodétecteurs.

Le procédé peut alors comporter :

- une sélection d'un premier couple source de lumière/photodétecteur dans le premier groupe;
- une sélection d'un deuxième couple source de lumière/photodétecteur dans le deuxième groupe.

Selon un mode de réalisation, le procédé comporte :

- une prise en compte d'une plage de validité de la pression artérielle ;
- lorsque la pression artérielle résultant de l'étape h) est située en dehors de la plage de validité, renouvellement de la phase de calibration.

Selon une possibilité, le critère de sélection acoustique est un rapport signal sur bruit maximal, ou une intensité maximale d'un signal acoustique détecté, la sélection de l'émetteur et du détecteur acoustique étant effectuée en fonction du signal acoustique, associé au couple émetteur acoustique/détecteur acoustique, dont le rapport signal sur bruit est maximal ou dont l'intensité est maximale.

Le critère de sélection optique peut comporter un critère de corrélation temporelle, à un décalage temporel près, de telle sorte que la sélection de chaque couple source-détecteur comporte :

- une estimation d'une corrélation temporelle entre les signaux détectés en différents instants par les photodétecteurs de chaque couple source de lumière-photodétecteur;
- une détermination des couples source de lumière-photodétecteur pour lesquels le signal résultant du photodétecteur présente la corrélation temporelle la plus élevée.

Le critère de sélection optique peut être un des critères de sélection décrits en lien avec le premier objet de l'invention.

Le procédé peut être tel que :

- le premier couple source de lumière - photodétecteur définit un premier point de mesure;
- le deuxième couple source de lumière - photodétecteur définit un deuxième point de mesure;
- le premier point de mesure et le deuxième point de mesure sont distants l'un de l'autre.

L'invention sera mieux comprise à la lecture de l'exposé des exemples de réalisation présentés, dans la suite de la description, en lien avec les figures listées ci-dessous.

## FIGURES

La figure 1 est un exemple de dispositif selon l'invention.
Les figures 2A à 2E illustrent une mise en œuvre de la modalité acoustique du dispositif.

Les figures 3A et 3B illustrent une mise en œuvre de la modalité optique du dispositif.
La figure 4 est un autre exemple de dispositif selon l'invention.
La figure 5 schématise les principales étapes de mise en œuvre d'un procédé de détermination d'une pression artérielle d'un utilisateur à l'aide d'un dispositif tel que décrit en lien avec la figure 1 ou la figure 4.

## EXPOSE DE MODES DE REALISATION PARTICULIERS

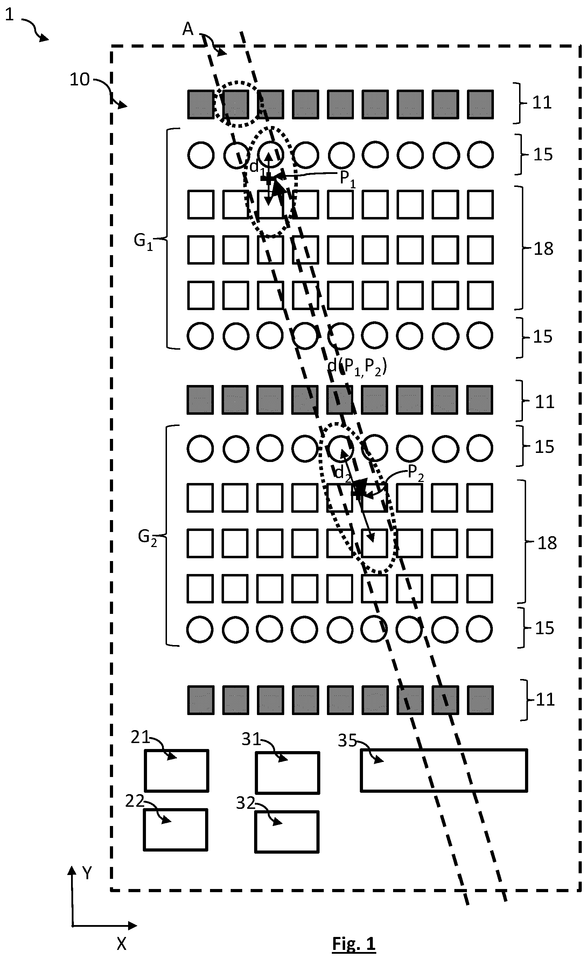

La figure 1 montre un exemple de dispositif 1 permettant une estimation de la pression artérielle d'un utilisateur. Le dispositif 1 comporte un support 10, destiné à être placé contre la peau d'un utilisateur, face à une artère A. Il peut par exemple s'agir de l'artère carotide, comme décrit dans [1] ou l'artère radiale pour un dispositif au poignet. Le support 10 est configuré pour être placé durablement au contact de la peau. Il s'agit de préférence d'un support souple, de façon à épouser le contour de la peau. Le support 10 peut être adhésif ou être relié à un moyen de maintien contre la peau, de type brassard ou bracelet élastique.

Le support comporte des composants permettant une mise en œuvre de modalités acoustiques ou optiques telles que décrites dans [1].

La modalité acoustique est mise en œuvre à l'aide de transducteurs acoustiques 11, répartis sur le support. Chaque transducteur acoustique est configuré pour émettre et/ou détecter une onde acoustique ultrasonore, de façon à déterminer une évolution temporelle du diamètre D(t) de l'artère A sous l'effet de l'activité cardiaque. Les transducteurs acoustiques peuvent être des transducteurs piézoélectriques ou des capteurs électromécaniques de type MEMS. Les transducteurs acoustiques sont reliés à une unité de traitement acoustique 31. De préférence, mais sans que cela ne soit nécessaire, chaque transducteur acoustique peut à la fois fonctionner en tant qu'émetteur acoustique ou détecteur acoustique.

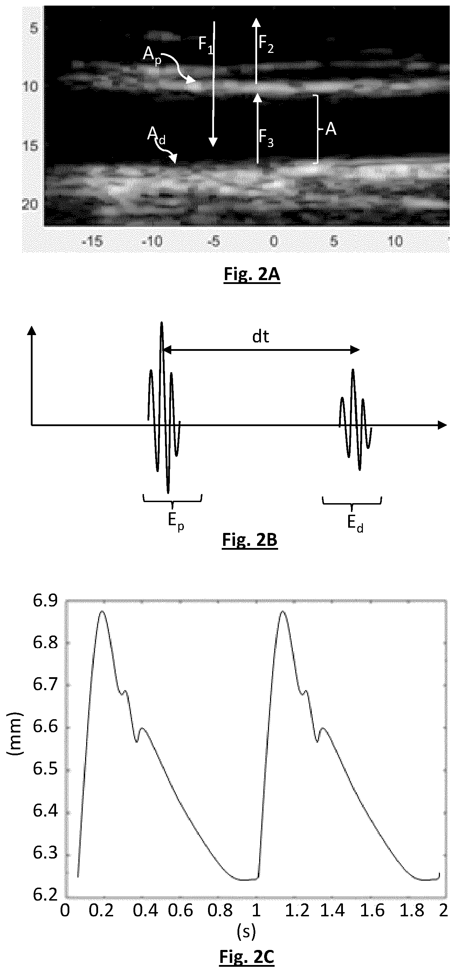

Le dispositif comporte une unité de traitement acoustique 31, configurée pour recevoir des signaux détectés par au moins un transducteur acoustique 11, fonctionnant en tant que détecteur acoustique, de façon à estimer, en différents instants, le diamètre de l'artère D(t). La modalité acoustique est décrite plus en détail en lien avec les figures 2A à 2E.

La modalité optique est mise en œuvre à l'aide de sources de lumière 15 et de photodétecteurs 18, répartis sur le support 10, et distants les uns des autres. Chaque source de lumière est configurée pour émettre une lumière vers la peau de l'utilisateur lorsqu'elle a été activée. Chaque photodétecteur est distant d'une source de lumière. Chaque photodétecteur est configuré pour détecter une lumière émanant de la peau de l'utilisateur suite à une activation d'au moins une source de lumière.

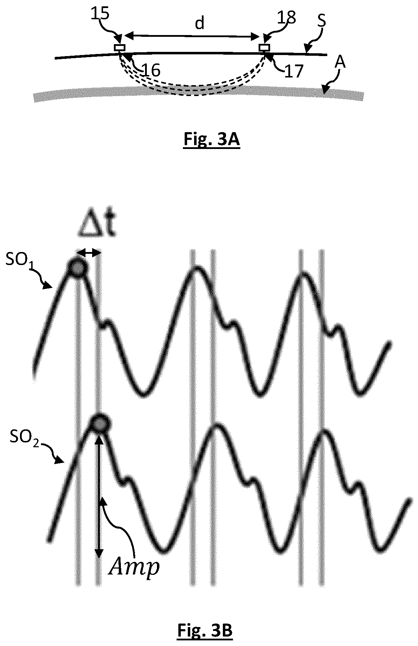

Chaque photodétecteur peut ainsi détecter une lumière émise par une source de lumière, et s'étant propagée à travers le corps de l'utilisateur, avant de ressortir de la peau de l'utilisateur, face au photodétecteur. Lorsque les photons détectés par un photodétecteur se sont propagés entre la peau et l'artère, la lumière détectée subit des variations périodiques sous l'effet de la variation périodique du volume sanguin dans les tissus sondés (artère, mais aussi veines, capillaires,...), induite par l'activité cardiaque. La modalité optique est décrite plus en détail en lien avec les figures 3A et 3B.

D'une façon générale, la modalité optique suppose la prise en compte de couples source- de lumière -photodétecteur, chaque couple associant une source de lumière et un photodétecteur, distant de la source de lumière. La distance entre une source de lumière et un photodétecteur d'un même couple peut être de l'ordre de quelques millimètres à quelques cm. Un photodétecteur (respectivement une source de lumière) peut former différents couples avec différentes sources de lumière (respectivement différents photodétecteurs).

Les photodétecteurs sont reliés à une unité de traitement optique 32. L'unité de traitement optique 32 est configurée pour recevoir des signaux détectés par au moins un photodétecteur 18, de façon à estimer, en différents instants, le délai mis par la perturbation du volume sanguin dans les tissus induite par l'activité cardiaque pour se propager entre deux points de mesure $P_1$, $P_2$ distants d'une distance de séparation $\Delta$, le long d'un segment artériel. Chaque point de mesure est située entre une source de lumière et un photodétecteur. La vitesse d'onde de pouls VOP est estimée à partir d'un ratio entre :

-     un décalage temporel $\Delta t$ entre les signaux respectivement détectés par chaque photodétecteur 18 ;
-     la distance de séparation $d(P_1, P_2)$ entre les deux points de mesure $P_1$ et $P_2$.

Le support 10 est disposé sur la peau de l'utilisateur, face à l'artère analysée A. Cependant, la position de l'artère par rapport au support 10 n'est pas connue avec précision, en particulier lorsque l'utilisateur est susceptible de bouger. Un aspect important de l'invention est de pouvoir sélectionner :

-     un émetteur et un détecteur acoustiques pertinents, c'est-à-dire correctement positionnés par rapport à l'artère A, de façon à obtenir une estimation correcte de la variation temporelle du diamètre de l'artère ;
-     deux couples source de lumière-photodétecteur pertinents, chaque couple comportant une source de lumière 15 et un photodétecteur 18, chaque couple étant positionné de façon à déterminer une variation du volume sanguin aux deux points de mesure $P_1$ et $P_2$. La comparaison des signaux issus de chaque photodétecteur permet une estimation de la vitesse de l'onde de pouls.

Le dispositif comporte une unité de sélection acoustique 21, reliée à chaque détecteur acoustique. L'unité de sélection acoustique 21 est programmée pour prendre en compte un critère de sélection acoustique et sélectionner un émetteur acoustique et un détecteur acoustique parmi les transducteurs acoustiques. L'émetteur et le détecteur acoustique sélectionnés sont ceux pour lesquels suite à l'émission d'une onde acoustique par l'émetteur acoustique, le détecteur acoustique détecte un signal acoustique considéré comme satisfaisant le plus au critère de sélection acoustique. Le critère de sélection acoustique est par exemple un rapport signal sur bruit maximal. Dans ce cas, le détecteur acoustique sélectionné par l'unité de sélection acoustique est par exemple celui qui génère, durant une période de mesure, le signal acoustique présentant le rapport signal sur bruit le plus élevé. Sur la figure 1, on a schématisé un transducteur acoustique, fonctionnant à la fois en tant qu'émetteur acoustique et en tant que détecteur acoustique, sélectionné par un contour en pointillés. Le transducteur acoustique sélectionné est centré (ou considéré comme tel) par rapport à l'artère A. Le signal résultant du transducteur sélectionné est ensuite transmis à l'unité de traitement acoustique 31 de façon à déterminer l'évolution temporelle D(t) du diamètre de l'artère.

Le dispositif comporte une unité de sélection optique 22, reliée à chaque photodétecteur 18. L'unité de sélection optique 22 est programmée pour prendre en compte un critère de sélection optique et sélectionner deux signaux optiques résultant des photodétecteurs respectifs de deux couples source de lumière-photodétecteur. Les couples sélectionnés sont ceux pour lesquels les photodétecteurs génèrent un signal optique considéré comme satisfaisant le plus au critère de sélection optique. Le critère de sélection optique est par exemple une corrélation entre les signaux respectivement générés par le photodétecteur de chaque couple. Dans ce cas, les couples source de lumière-photodétecteur sélectionnés par l'unité de sélection optique 22 sont ceux générant des signaux optiques temporellement corrélés, au décalage temporel près. Par temporellement corrélé, on entend que les évolutions, en fonction du temps, des signaux respectivement aux deux points de mesure, sont corrélées. Le décalage temporel dépend de la distance entre les points de mesure respectivement définis par chaque couple source de lumière

-     photodétecteur. Sur la figure 1, on a schématisé deux couples source de lumière-photodétecteurs sélectionnés par deux contours en pointillés. On considère que chaque point de mesure $P_1$ et $P_2$ est situé face au milieu d'un segment de droite reliant la source de lumière au photodétecteur du couple source de lumière-photodétecteur sélectionné. Sur la figure 1, on a représenté :

  -     une double flèche matérialisant une première distance, dite de rétrodiffusion, $d_1$ entre la

source et le photodétecteur du premier couple source de lumière-photodétecteur sélectionné ;

- une double flèche matérialisant une deuxième distance de rétrodiffusion $d_2$ entre la source et le photodétecteur du deuxième couple source de lumière-photodétecteur sélectionné.

De préférence, chaque couple source de lumière-photodétecteur sélectionné s'étend de part et d'autre de l'artère, ou le long de l'artère.

Les points de mesure $P_1$ et $P_2$ respectivement formés par les deux couples source de lumière - photodétecteur sélectionnés sont espacés l'un de l'autre. La distance $d(P_1, P_2)$, le long de l'artère, entre les deux points de mesure, est de préférence supérieure à 1 cm ou à 5 cm.

Les signaux résultant des photodétecteurs de chaque couple source de lumière-photodétecteur sélectionné sont adressés à l'unité de traitement optique 32, de façon à estimer la vitesse d'onde de pouls VOP.

De préférence, les sources de lumière 15 et les photodétecteurs 18 sont répartis sur le support 10 en formant un premier groupe $G_1$ et un deuxième groupe $G_2$. L'unité de sélection optique 22 est programmée pour sélectionner :

- un premier couple source de lumière-photodétecteur parmi les sources de lumière 15 et photodétecteurs 18 du premier groupe $G_1$;
- un deuxième couple source de lumière-photodétecteur parmi les sources de lumière 15 et photodétecteurs 18 du deuxième groupe $G_2$.

Cette répartition selon deux groupes permet de garantir une distance de séparation minimale $d(P_1, P_2)_{min}$ entre les premier et deuxième points de mesure $P_1$, $P_2$ respectivement définis par chaque couple source de lumière-photodétecteur sélectionné. La distance $d(P_1,P_2)$ entre les points de mesure $P_1$ et $P_2$ est supposée connue par la connaissance de la géométrie du dispositif. Elle est représentée sur la figure 1 par une double flèche en pointillés.

Les figures 2A, 2B et 2C illustrent le fonctionnement de la modalité acoustique. Chaque transducteur émet une onde acoustique incidente, symbolisée par la flèche $F_1$. Cette dernière se propage à travers la peau de l'utilisateur, vers l'artère A. Une partie de l'onde acoustique incidente est successivement réfléchie par une portion proximale $A_p$ et une portion distale $A_d$, diamétralement opposées, de la paroi de l'artère A. Sur la figure 2A, les flèches $F_2$ et $F_3$ correspondent respectivement aux parties de l'onde incidente respectivement réfléchies par les portions proximale $A_p$ et distale $A_d$. Le transducteur (ou un autre transducteur) détecte une les ondes successivement réfléchies par les portions proximales $A_p$ et distale $A_d$, ces dernières formant respectivement un écho proximal $E_p$ et un écho distal $E_d$. La figure 2B schématise les échos. Sur la figure 2B, l'axe des ordonnées correspondant à l'amplitude de l'onde détectée et l'axe des abscisses correspond au temps. Le décalage temporel dt entre les deux échos $E_p$ et $E_d$ permet d'estimer le diamètre de l'artère, connaissant la vitesse de propagation de l'onde acoustique. Les mesures représentées sur la figure 2B schématisent un signal acoustique adressé par chaque transducteur 11 à l'unité de sélection 21.

Les mesures schématisées sur la figure 2B sont répétées en différents instants de mesure, selon une fréquence d'échantillonnage pouvant être par exemple de 1 kHz. La figure 2C représente une évolution temporelle du diamètre D(t) de l'artère (axe des ordonnées - unités $\mu$m) en fonction du temps t (unité : seconde). Le diamètre maximal correspond à la systole, tandis que le diamètre minimal correspond à la diastole. Dans cet exemple, la variation de diamètre $\Delta$D entre la systole et la diastole est de l'ordre de 0.6 mm.

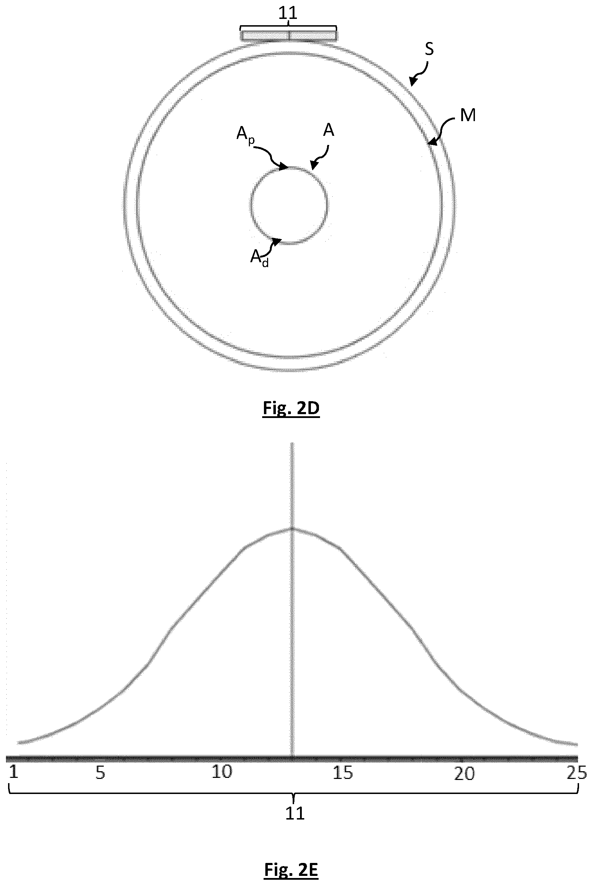

La position du transducteur 11 par rapport à l'artère a une influence sur la qualité de la mesure de D(t). Des simulations ont montré que lorsque le transducteur n'est pas centré par rapport à l'artère, l'intensité de l'onde acoustique réfléchie diminue. Compte tenu du bruit de mesure, cela augmente l'incertitude dans la détermination de l'intervalle temporel dt, et donc de l'évolution temporelle D(t). La figure 2D montre une configuration de simulation. On a simulé une sonde acoustique comportant une ligne de 25 transducteurs 11, chacun fonctionnant en mode émetteur/détecteur, disposée au contact d'une peau S, de forme cylindrique, et de rayon environ 13 mm. L'artère A a été modélisée au centre du cylindre modélisant la peau S. Entre la peau et l'artère s'étend un milieu M correspondant à un muscle. Ainsi, l'artère A est située à une profondeur comprise entre 10 mm et 15 mm sous la peau S, ce qui correspond à une profondeur d'artère usuelle. A l'aide du logiciel CIVA (fournisseur Extende), on a évalué l'intensité des échos produits par chaque transducteur en réponse à une impulsion acoustique incidente. Les densités et vitesses de propagation acoustique dans les milieux modélisés S, M et A sont représentatives de celles du sang, des muscles et de la peau respectivement. La figure 2E représente l'évolution de l'amplitude du signal (axe des ordonnées - unités arbitraires) en fonction du transducteur (axe des abscisses). Dans ce cas, l'émission et la réception sont réalisées par le même transducteur. Les transducteurs 1, 13 et 25 correspondent respectivement aux positions extrême gauche, centrée et extrême droite de la sonde. On observe qu'une position centrée du transducteur acoustique, par rapport à l'artère, permet d'obtenir une intensité d'écho maximale. Ainsi, la position du transducteur a une influence importante sur la qualité de la mesure effectuée. L'unité de sélection acoustique 21 permet une sélection du transducteur acoustique générant les échos dont le signal est maximal parmi les autres transducteurs. Il en résulte une estimation précise de l'évolution temporelle D(t) du diamètre D de l'artère.

Selon une possibilité, plusieurs transducteurs, déphasés les uns des autres, sont utilisés simultanément. L'unité de sélection permet de sélectionner un transducteur de référence, puis d'estimer un déphasage temporel entre les différents transducteurs par rapport au transducteur de référence.

Les figures 3A et 3B illustrent le fonctionnement de la modalité optique. On a représenté une source de lumière 15 et un photodétecteur 18 formant un couple source de lumière-photodétecteur. La source de lumière 15 émet un faisceau lumineux incident 16 se propageant, à travers la peau, vers l'artère A de l'utilisateur. La direction d'émission est généralement perpendiculaire à la peau. Le faisceau lumineux est de préférence émis selon une bande spectrale d'émission étroite, de préférence < 50 nm, dans une plage spectrale comprise entre 500 nm et 1200 nm, et de préférence entre 500 nm et 1000 nm. Cette plage spectrale correspond à une absorption significative de la lumière par l'hémoglobine. Les bandes spectrales peuvent être centrées sur les longueurs d'onde suivantes : 525 nm, 660 nm, 740 nm, 805 nm, 850 nm.

Chaque source de lumière peut être une LED (diode électroluminescente), ou une extrémité d'une fibre optique, dont l'autre extrémité est disposée face à une source lumineuse. Chaque photodétecteur peut être une photodiode, ou une extrémité lumineuse d'une fibre optique, dont l'autre extrémité est reliée à un capteur de lumière. De façon alternative, chaque source de lumière peut être une diode laser, un VECSEL ou l'extrémité d'un guide de lumière, par exemple une fibre optique.

Les photons du faisceau lumineux incident 16 pénètrent dans le corps de l'utilisateur. Ils se propagent dans les tissus entre la peau et l'artère A, cette dernière étant située à une profondeur, sous la peau, de l'ordre de 10 mm. Une partie des photons incidents est rétrodiffusée selon une direction parallèle à la direction d'émission. Les photons rétrodiffusés constituent un rayonnement rétrodiffusé 17. Le rayonnement rétrodiffusé 17 peut être détecté par le photodétecteur 18, placé en regard de la peau de l'utilisateur. La distance d entre la source de lumière et le photodétecteur, dite distance de rétrodiffusion, est généralement non nulle et est généralement comprise entre 5 mm et quelques cm . Le photodétecteur 18 permet ainsi de mesurer l'intensité du rayonnement rétrodiffusé selon la distance de rétrodiffusion d. Sur la figure 3A, les flèches courbes en pointillés représentent des trajets optiques de photons émis par la source de lumière 15 et détectés par le photodétecteur 18. Le photodétecteur est ainsi agencé pour mesurer une intensité d'un faisceau de lumière formé par des photons rétrodiffusés.

Plus la distance de rétrodiffusion est élevée, plus les photons constituant le rayonnement rétrodiffusé 17 pénètrent les tissus de l'utilisateur selon une profondeur élevée. L'intensité du rayonnement rétrodiffusé dépend de la variation de volume sanguin dans l'artère. Plus la quantité de sang est importante, plus la quantité de photons absorbés par l'hémoglobine est importante, et plus l'intensité du rayonnement rétrodiffusé diminue. A chaque battement cardiaque, l'afflux sanguin entraîne, dans les tissus sondés, une modulation de l'absorption de la lumière se propageant dans les tissus. Il en résulte une modulation de l'intensité du rayonnement rétrodiffusé détecté par le photodétecteur 18 associé à la source de lumière.

Le signal détecté par le photodétecteur 18 comporte une composante continue, à laquelle s'ajoute une composante pulsatile, cette dernière variant en fonction de l'activité cardiaque. L'intensité détectée par le photodétecteur comporte ainsi une composante périodique, dont la fréquence fondamentale correspond à la fréquence cardiaque.

Comme représenté sur la figure 1, le dispositif permet de sélectionner deux couples source de lumière-photodétecteur, définissant respectivement deux points de mesure $P_1$ et $P_2$, le long de l'artère, espacés d'une distance de séparation $d(P_1, P_2)$. Du fait de la distance de séparation, les signaux périodiques résultant respectivement du premier et du deuxième couples source de lumière-photodétecteur sont décalés d'un décalage temporel $\Delta t$. Connaissant la distance de séparation $d(P_1, P_2)$, l'estimation du décalage temporel , appelé temps de transit de l'onde de pouls, permet une estimation d'une vitesse, dite « vitesse d'onde de pouls » (VOP), selon l'expression :

$$VOP = \frac{d(P_1, P_2)}{\Delta t} \quad (1)$$

La figure 3B représente :

- un premier signal optique $SO_1$ résultant du photodétecteur du premier couple source de lumière-photodétecteur ;
- un deuxième signal optique $SO_2$ résultant du photodétecteur du deuxième couple source de lumière-photodétecteur.

Sur la figure 3B, chaque signal correspond à l'opposé du signal détecté : ainsi, chaque signal $SO_1$, $SO_2$ est représentatif de l'absorption périodique des tissus aux points de mesure $P_1$ et $P_2$. Le décalage temporel peut être estimé à partir de l'écart temporel $\Delta t$ entre des points remarquables de chaque courbe, par exemple des maximas ou des minimas.

De préférence la distance de séparation $d(P_1, P_2)$ est supérieure à 5 mm . Elle est de préférence inférieure à 10 cm ou 5 cm.

La fréquence d'acquisition des signaux résultant de chaque photodétecteur peut être comprise entre 100 Hz et 100 kHz, ce qui permet une estimation de la vitesse d'onde de pouls avec une résolution temporelle suffisante.

Cependant, la profondeur de l'artère sous la peau n'est pas connue avec précision. De même, la position de l'artère, parallèlement au support 10, n'est pas connue, ou peut varier en fonction des mouvements de l'utilisateur. Ainsi, il est difficile de déterminer a priori les couples source-photodétecteurs les plus pertinents pour estimer précisément la vitesse d'onde de pouls. On comprend, de la figure 3A, que la distance de rétrodiffusion doit être suffisante pour permettre une propagation des photons détectés à travers l'artère ou un volume de tissu impacté par la perturbation du volume sanguin induite par l'activité cardiaque. Il est également préférable que la source et le photodétecteur d'un même couple soient disposés soit le long de l'artère, soit de part et d'autre de l'artère, de façon que le point de mesure, situé entre la source et le photodétecteur, soit situé proche de l'artère.

L'unité de sélection optique 22 prend en compte différents signaux détectés tels que représentés sur la figure 3B. Il s'agit de signaux optiques représentatifs de l'intensité détectée par les photodétecteurs de différents couples sources de lumière-photodétecteurs. Les signaux détectés comportent un composante, dite pulsatile, pouvant être considérée comme périodique, du fait de la répétition des cycles cardiaques. La sélection des couples sources-photodétecteur pertinents est effectuée en prenant en compte un critère de sélection optique et en déterminant deux couples sources de lumière-photodétecteurs pour lesquels le critère de sélection optique est satisfait.

Le critère de sélection optique peut être une corrélation temporelle. En effet, les signaux détectés les plus pertinents ont une évolution temporelle comparable, qui correspond à l'activité cardiaque. Le fait de sélectionner des signaux détectés ayant une corrélation temporelle élevée facilite la détermination du décalage temporel $\Delta t$ illustré sur la figure 3B. Par corrélation temporelle, on entend une corrélation à un décalage temporel près qui correspond à la distance entre les points de mesure le long de l'artère.

D'autres critères de sélection optique peuvent être appliqués en compléments ou en remplacement du critère de corrélation. Au cours de chaque période, la composante pulsatile de chaque signal détecté décrit une oscillation d'une certaine amplitude *Amp.* Le critère d'acceptation peut être une amplitude supérieure à un certain seuil, ou située dans une plage d'acceptation préalablement définie.

Le critère de sélection optique peut être une corrélation de l'évolution temporelle de chaque signal détecté, au cours d'une période, avec une forme prédéterminée. On sélectionne alors deux couples source de lumière-photodétecteur présentant la meilleure corrélation avec une forme temporelle prédéterminée.

Un autre critère de sélection optique peut être une distance minimale entre la source et le photodétecteur formant un couple de source de lumière-photodétecteur, de façon que la profondeur adressée soit suffisante.

Les critères de sélection optiques précédemment décrits peuvent être combinés.

L'unité de traitement optique 32 estime ensuite la vitesse d'onde de pouls à partir signaux optiques générés par les photodétecteurs des couples sources de lumière-photo détecteurs sélectionnés.

Le dispositif comporte une unité de calcul 35, configurée pour estimer une valeur de pression artérielle à partir de l'évolution temporelle de diamètre D(t) résultant de l'unité de traitement acoustique 31 et de la VOP résultant de l'unité de traitement optique 32. La pression artérielle P est telle que

$$P = f(D, VOP) \quad (2)$$

où f est une fonction préalablement définie.

Par exemple, la fonction *f* peut être telle que :

$$P(t) - P_0 = 2\rho(VOP)^2 \ln\left(\frac{D(t)}{D_0}\right) \quad (3)$$

où :

- $P(t)$ est la pression artérielle à un instant $t$
- $D_0$ est un diamètre de référence, par exemple le diamètre en fin de diastole
- $P_0$ est une pression de référence correspondant à $D = D_0$.

La pression artérielle $P(t)$ obtenue par l'équation (3) décrit une fonction périodique, chaque période correspondant à un battement cardiaque. Durant chaque période, la pression maximale correspond à la pression artérielle systolique, la pression minimale correspond à la pression artérielle diastolique. La pression artérielle moyenne est la moyenne de la pression sur une période.

La fonction *f* précédemment évoquée peut être déterminée par une calibration, en présence d'une mesure de référence de la pression artérielle de l'utilisateur. On peut alors établir un lien entre les valeurs mesurées (D, VOP) en différents instants avec une pression artérielle de référence mesurée par la méthode de référence. La confrontation entre les valeurs mesurées et la pression de référence mesurée permet d'établir la fonction de calibration.

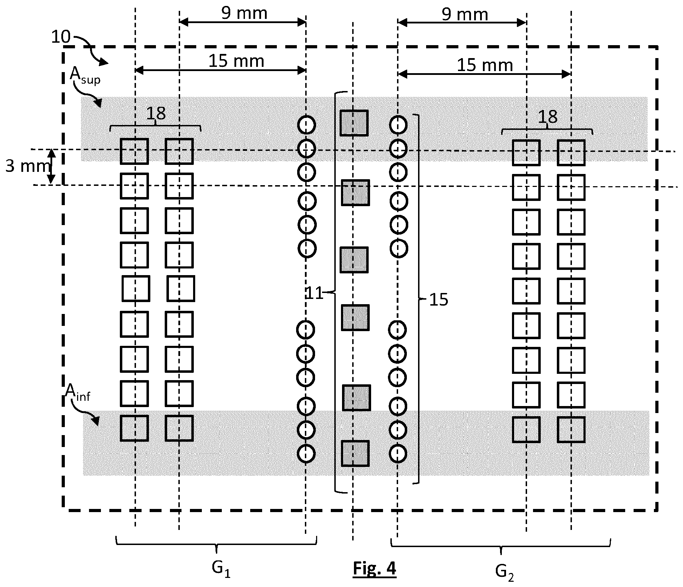

La figure 4 représente un autre exemple de dispositif convenant à la mise en œuvre de l'invention. On a indiqué, sur la figure 4, les principales dimensions. Le dispositif permet l'analyse d'une artère A s'étendant entre les deux positions extrêmes représentées $A_{sup}$ et $A_{inf}$ représentées sur la figure 4. On a représenté, par une accolade, les deux groupes $G_1$ et $G_2$, dans lesquels les deux couples source de lumière-photodétecteur sont à identifier en fonction de la position de l'artère par rapport au support 10.

Selon une variante, le support 10 est divisé en deux supports élémentaires distants l'un de l'autre. Le premier support élémentaire comporte un premier groupe $G_1$ de photodétecteurs et de source de lumière. Un deuxième support élémentaire comporte un deuxième groupe de photodétecteurs et de sources de lumière. les transducteurs peuvent être fixés sur un des supports élémentaires ou sur les deux supports élémentaires. Ainsi, le support 10 peut ne pas être monolithique et comporter différents supports élémentaires.

Les unités de sélection acoustique ou optique, ainsi que l'unité centrale, peuvent former une même unité, mise en œuvre par un microprocesseur. De façon alternative, chacune de ces unités met en œuvre un microprocesseur. Selon une possibilité, tout ou partie des unités de sélection ou de l'unité centrale est déportée, à distance du support 10. Le dispositif comporte une unité de transmission de façon à transmettre les signaux issus des transducteurs et des photodétecteurs par liaison filaire ou sans fil.

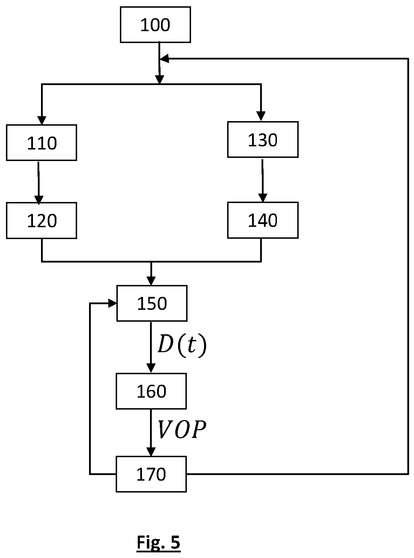

La figure 5 schématise les principales étapes d'un procédé mettant en œuvre un dispositif tel que représenté sur les figures 1 ou 4.

Etape 100 : disposition du support sur la peau d'un utilisateur, face à une artère.

Etape 110 : émission d'une onde acoustique par un émetteur acoustique et détection d'une onde acoustique réfléchie par un détecteur acoustique, l'étape 110 étant répétée pour différents émetteurs acoustiques et/ou différents détecteurs acoustiques. Chaque signal acoustique détecté est susceptible de comporter des échos représentatifs de la réflexion, par l'artère, de l'onde acoustique émise par l'émetteur acoustique.

Etape 120 : traitement des signaux acoustiques par l'unité de sélection acoustique, de façon à identifier le couple émetteur / détecteur acoustique pertinent. Ce dernier correspond au détecteur dont le signal acoustique détecté, suite à l'émission d'une onde acoustique par l'émetteur acoustique, satisfait le plus au critère de sélection acoustique précédemment évoqué.

Etape 130 : acquisition de signaux optiques par les différents photodétecteurs de différents couples source de lumière-photodétecteur. Lors de cette étape, on peut activer séquentiellement une source de lumière et on acquiert des signaux optiques issus de différents photodétecteurs considérés comme suffisamment proches de la source pour détecter un rayonnement rétrodiffusé exploitable. Les sources de lumière sont activées successivement. On obtient ensuite des signaux optiques pour chaque couple source de lumière - photodétecteur.

Etape 140 : Traitement des signaux optiques résultant de chaque photodétecteur, lors de l'étape 130 par l'unité de sélection optique, de façon à identifier les deux couples source de lumière-photodétecteur pertinents. Ces derniers correspondent aux couples dont les signaux, résultant du photodétecteur, satisfont le plus au critère de sélection optique.

Les étapes 110 à 140 correspondent à une phase de calibration, sur la base des mesures de calibration acquises lors des étapes 110 et 130. L'acquisition des mesures de calibration peut être effectuées durant une période de calibration prédéfinie, pouvant durer quelques minutes par exemple.

Les étapes 150 à 170 correspondent à la mise en œuvre du dispositif, suite à la phase de calibration.

Etape 150 : détermination de l'évolution temporelle du diamètre D(t) de l'artère, en mettant en œuvre la modalité acoustique. Au cours de cette étape, l'unité de traitement acoustique 31 reçoit le signal acoustique résultant du détecteur acoustique sélectionné lors de l'étape 120.

Etape 160 : détermination de l'évolution la vitesse d'onde de pouls VOP de l'artère, en mettant en œuvre la modalité optique. Au cours de cette étape, l'unité de traitement optique 32 reçoit les signaux optiques résultant des photodétecteurs respectifs des deux couples source de lumière-photodétecteur sélectionnés lors de l'étape 140.

Les étapes 150 et 160 peuvent être effectués simultanément ou dans un ordre quelconque.

Etape 170 : estimation de la pression artérielle en fonction du diamètre de l'artère D(t) et de la VOP résultant des étapes 150 et 160, par exemple en utilisant l'expression (3) ou une autre fonction de calibration

Les étapes 150 à 170 peuvent être réitérées à des instants de mesure successifs, de façon à effectuer une mesure de la pression artérielle « en continu », c'est-à-dire à des instants suffisamment rapprochés. La phase de calibration (étapes 110 à 140) peut être réitérée périodiquement, de façon à identifier les transducteurs acoustiques et couples source de lumière/photodétecteur les plus pertinents. La phase de calibration peut également être effectuée en cas de survenue d'une mesure de pression artérielle considérée comme anormale.

L'invention tire profit du fait que le dispositif permet l'estimation d'une pression artérielle sans recours à une compression d'un membre de l'utilisateur. Elle est robuste à des incertitudes de positionnement du support du dispositif sur la peau de l'utilisateur, ainsi qu'à des éventuels mouvements de l'utilisateur portant le support.

**Revendications**

1. Dispositif d'estimation d'une pression artérielle d'un utilisateur, le dispositif étant destiné à être porté par l'utilisateur, le dispositif comportant :

   - un support (10), destiné à être appliqué contre la peau de l'utilisateur ;
   - plusieurs sources de lumière (15), disposées sur le support, configurées pour émettre une lumière (16) vers la peau de l'utilisateur lorsqu'elles sont activées ;
   - plusieurs photodétecteurs (18), disposés sur le support, distants de chaque source de lumière, configurés pour détecter une lumière (17) émanant de la peau de l'utilisateur suite à une activation d'au moins une source de lumière, chaque photodétecteur formant, avec ladite source de lumière, un couple source-photodétecteur ;

- plusieurs transducteurs acoustiques (11), chacun comportant au moins :

> • un émetteur acoustique, configuré pour émettre une onde acoustique à travers la peau ;
> • et un détecteur acoustique, configuré pour détecter une onde acoustique réfléchie dans le corps de l'utilisateur, se propageant à travers la peau ;

- une unité de sélection acoustique (21), programmée pour :

> • prendre en compte un critère de sélection acoustique ;
> • sélectionner, selon le critère de sélection acoustique, un émetteur acoustique et un détecteur acoustique parmi les transducteurs acoustique, la sélection étant effectuée en fonction d'un signal acoustique détecté par chaque détecteur acoustique suite à une émission d'une onde acoustique par au moins un émetteur acoustique;

- une unité de sélection optique (22), configurée pour :

> • prendre en compte un critère de sélection optique ;
> • sélectionner, selon le critère de sélection optique, un premier couple source de lumière - photodétecteur, comportant une première source de lumière et un premier photodétecteur, choisis parmi les sources de lumière et les photodétecteurs, et un deuxième couple source de lumière - photodétecteur, comportant une deuxième source de lumière et un deuxième photodétecteur, choisis parmi les sources de lumière et les photodétecteurs, la sélection étant effectuée en fonction des signaux détectés par le premier photodétecteur et le deuxième photodétecteur suite à une activation de la première source de lumière et de la deuxième source de lumière ;

- une unité centrale (35), programmée pour:

> • calculer un décalage temporel entre deux échos dans le signal détecté par le détecteur acoustique sélectionné;
• calculer un décalage temporel entre des signaux détectés par le premier photodétecteur et le deuxième photodétecteur ;
• estimer une pression artérielle à partir des décalages temporels calculés ;

**2.** Dispositif selon la revendication 1, dans lequel l'unité centrale est programmée pour :

> • estimer un diamètre artériel (D(t)) à partir du décalage temporel entre les échos du signal détecté par le détecteur acoustique sélectionné ;
> • estimer une vitesse d'onde de pouls (VOP), à partir du décalage temporel entre les signaux détectés par le premier photodétecteur et le deuxième photodétecteur ;
> • estimer la pression artérielle (P(t)), en fonction du diamètre artériel et la vitesse d'onde de pouls estimés.

**3.** Dispositif selon l'une quelconque des revendications 1 ou 2, dans lequel chaque source de lumière émet une lumière dans une bande spectrale comprise entre 500 nm et 1200 nm.

**4.** Dispositif selon l'une quelconque des revendications précédentes, comportant

> - un premier groupe ($G_1$) de sources de lumière et de photodétecteurs ;
> - un deuxième groupe ($G_2$) de sources de lumière et de photodétecteurs, distant du premier groupe de sources de lumière et de photodétecteurs ;
> - l'unité de sélection optique est configurée pour
>
> > • sélectionner la première source de lumière et le premier photodétecteur parmi les sources de lumière et les photodétecteurs du premier groupe ;
> > • sélectionner la deuxième source de lumière et le deuxième photodétecteur parmi les sources de lumière et les photodétecteurs du deuxième groupe.

**5.** Dispositif selon l'une quelconque des revendications précédentes, dans lequel le critère de sélection acoustique étant un rapport signal sur bruit, l'unité de sélection acoustique est configurée pour :

> - estimer un rapport signal sur bruit de chaque signal détecté par d'un détecteur acoustique ;
> - sélectionner le détecteur acoustique pour lequel le rapport signal sur bruit est le plus élevé.

**6.** Dispositif selon l'une quelconque des revendications précédentes, dans lequel le critère de sélection optique étant un critère de corrélation, l'unité de sélection optique est configurée pour:

- estimer une corrélation temporelle entre les signaux détectés en différents instants par des photodétecteurs de chaque couple source de lumière-photodétecteur;
- sélectionner la première source de lumière et le premier photodétecteur ainsi que la deuxième source de lumière et le deuxième photodétecteur en fonction de la corrélation temporelle estimée.

7. Dispositif selon l'une quelconque des revendications 1 à 6, dans lequel le critère de sélection optique étant un critère d'amplitude, l'unité de sélection optique est configurée pour :

- estimer une amplitude d'une évolution temporelle de signaux détectés en différents instants par des photodétecteurs de chaque couple source de lumière-photodétecteur;
- sélectionner la première source de lumière et le premier photodétecteur ainsi que la deuxième source de lumière et le deuxième photodétecteur en fonction de l'amplitude.

8. Dispositif selon l'une quelconque des revendications précédentes, dans lequel le critère de sélection optique étant un critère de forme, l'unité de sélection optique est configurée pour:

- prendre en compte une forme temporelle prédéterminée ;
- déterminer une évolution temporelle des signaux détectés, en différents instants, par les photodétecteurs de chaque couple source de lumière-photodétecteur;
- sélectionner la première source de lumière et le premier photodétecteur ainsi que la deuxième source de lumière et le deuxième photodétecteur en fonction d'une corrélation entre l'évolution temporelle des signaux détectés et la forme temporelle prédéterminée.

9. Procédé d'estimation d'une pression artérielle à l'aide d'un dispositif selon l'une quelconque des revendications précédentes, le procédé comportant :

a) disposition du support (10) sur la peau d'un utilisateur, face à une artère (A) ;
b) émission d'au moins une onde acoustique incidente par un émetteur acoustique et acquisition de signaux acoustiques par un détecteur acoustique, chaque signal acoustique détecté comportant des échos représentatifs de réflexions de l'onde acoustique incidente par l'artère, l'étape b) étant réalisée pour différents émetteurs acoustique et/ou différents détecteurs acoustiques, de telle sorte que chaque signal acoustique détecté est associé à un émetteur acoustique et à un détecteur acoustique ;
c) à l'aide de l'unité de sélection acoustique :

- prise en compte d'un critère de sélection acoustique ;
- sélection d'un émetteur acoustique et d'un détecteur acoustique, en fonction d'une confrontation entre chaque signal acoustique détecté, lors de l'étape b), et le critère de sélection acoustique ;

d) pour chaque source de lumière, émission d'une lumière incidente vers la peau de l'utilisateur et détection d'un rayonnement rétrodiffusé par au moins un photodétecteur, chaque photodétecteur générant un signal optique représentatif de l'intensité du rayonnement rétrodiffusé ;
e) à l'aide de l'unité de sélection optique :

- prise en compte d'un critère de sélection optique ;
- sélection de deux couples source de lumière - photodétecteur, chaque couple comportant une source de lumière et un photodétecteur, en fonction d'une confrontation entre chaque signal optique issu de chaque photodétecteur et du critère de sélection optique ;

f) émission d'une onde acoustique incidente par le transducteur acoustique sélectionné lors de c), et formation d'un signal acoustique représentatif d'échos suite à des réflexions de l'onde acoustique incidente par l'artère ;
g) activation des sources de lumière de chaque couple source de lumière-photodétecteur sélectionné lors de e) et formation, par chaque photodétecteur de chaque couple, d'un signal optique représentatif de l'intensité du rayonnement rétrodiffusé par l'artère ;
h) en fonction d'un décalage temporel entre deux échos du signal acoustique, et d'un décalage temporel entre les signaux optiques formés par chaque photodétecteur, estimation de la pression artérielle de l'utilisateur.

10. Procédé selon la revendication 9, dans lequel l'étape h) comporte :

- h1) en fonction du signal acoustique formé, estimation du diamètre de l'artère;
- h2) en fonction des signaux optiques formés, en différents instants, par chaque photodétecteur sélectionné, estimation d'une vitesse d'onde de pouls ;

- h3) à partir du diamètre de l'artère résultant de la sous-étape h1) et de la vitesse d'onde de pouls résultant de la sous-étape h2), estimation d'une pression artérielle de l'utilisateur.

11. Procédé selon la revendication 10, dans lequel la sous-étape h2) comporte une estimation d'un décalage temporel ($\Delta t$) entre les signaux optiques respectivement formés par le premier photodétecteur et le deuxième photodétecteur.

12. Procédé selon l'une quelconque des revendications 9 à 11, dans lequel les étapes a) à e) constituent une phase de calibration du dispositif, les étapes f) à h) étant réitérées entre deux calibrations successives.

13. Procédé selon l'une quelconque des revendications 9 à 12, dans lequel le dispositif est un dispositif selon la revendication 4, le procédé comportant :

   - une sélection d'un premier couple source de lumière/photodétecteur dans le premier groupe ($G_1$);
   - une sélection d'un deuxième couple source de lumière/photodétecteur dans le deuxième groupe ($G_2$).

14. Procédé selon l'une quelconque des revendications 9 à 12, comportant

   - une prise en compte d'une plage de validité de la pression artérielle ;
   - lorsque la pression artérielle résultant de l'étape h) est située en dehors de la plage de validité, renouvellement de la phase de calibration.

15. Procédé selon l'une quelconque des revendications 9 à 14, dans lequel le critère de sélection acoustique est un rapport signal sur bruit maximal, la sélection de l'émetteur et du détecteur acoustique étant effectuée en fonction du signal acoustique, associé au couple émetteur acoustique/détecteur acoustique, dont le rapport signal sur bruit est maximal.

16. Procédé selon l'une quelconque des revendications 9 à 15, dans lequel le critère de sélection optique comporte un critère de corrélation temporelle, à un décalage temporel près, de telle sorte que la sélection de chaque couple source-détecteur comporte :

   - une estimation d'une corrélation temporelle entre les signaux détectés par les photodétecteurs de chaque couple source de lumière-photodétecteur en différents instants;
   - une détermination des couples source de lumière-photodétecteur pour lesquels le signal résultant du photodétecteur présente la corrélation temporelle la plus élevée.

17. Procédé selon l'une quelconque des revendications 9 à 16, dans lequel :

   - le premier couple source de lumière - photodétecteur définit un premier point de mesure ($P_1$) ;
   - le deuxième couple source de lumière - photodétecteur définit un deuxième point de mesure ($P_2$) ;
   - le premier point de mesure et le deuxième point de mesure sont distants l'un de l'autre.

**Patentansprüche**

1. Vorrichtung zur Schätzung eines arteriellen Drucks eines Benutzers, wobei die Vorrichtung dazu bestimmt ist, von dem Benutzer getragen zu werden, wobei die Vorrichtung umfasst:

   - einen Träger (10), der dazu bestimmt ist, an die Haut des Benutzers angelegt zu werden;
   - mehrere Lichtquellen (15), die auf dem Träger angeordnet sind und dazu ausgestaltet sind, ein Licht (16) zu der Haut des Benutzers hin auszusenden, wenn sie aktiviert sind;
   - mehrere Fotodetektoren (18), die auf dem Träger angeordnet sind, von jeder Lichtquelle entfernt sind und dazu ausgestaltet sind, ein nach einer Aktivierung mindestens einer Lichtquelle von der Haut des Benutzers ausgehendes Licht (17) zu detektieren, wobei jeder Fotodetektor mit der Lichtquelle ein Quelle-Fotodetektor-Paar bildet;
   - mehrere akustische Wandler (11), wobei jeder mindestens umfasst:

      • einen akustischen Sender, der dazu ausgestaltet ist, eine akustische Welle durch die Haut auszusenden;
      • und einen akustischen Detektor , der dazu ausgestaltet ist, eine in dem Körper des Benutzers reflektierte akustische Welle, die sich durch die Haut ausbreitet, zu detektieren;

   - eine akustische Auswahleinheit (21), die dazu programmiert ist:

      • ein akustisches Auswahlkriterium zu berücksichtigen;
      • entsprechend dem akustischen Auswahlkriterium einen akustischen Sender und einen akustischen Detektor unter den akustischen Wandlern auszuwählen, wobei das Auswählen in Abhängigkeit von einem akustischen Signal durchgeführt wird, das von jedem akustischen Detektor nach einem Aussenden einer akustischen Welle durch mindestens einen akustischen Sender detektiert wird;

- eine optische Auswahleinheit (22), die dazu ausgestaltet ist:

    • ein optisches Auswahlkriterium zu berücksichtigen;
    • entsprechend dem optischen Auswahlkriterium ein erstes Lichtquelle/Fotodetektor-Paar, das eine erste Lichtquelle und einen ersten Fotodetektor umfasst, die unter den Lichtquellen und den Fotodetektoren ausgewählt sind, und ein zweites Lichtquelle/-Fotodetektor-Paar, das eine zweite Lichtquelle und einen zweiten Fotodetektor umfasst, die unter den Lichtquellen und den Fotodetektoren ausgewählt sind, auszuwählen, wobei das Auswählen in Abhängigkeit von den Signalen durchgeführt wird, die von dem ersten Fotodetektor und dem zweiten Fotodetektor nach einer Aktivierung der ersten Lichtquelle und der zweiten Lichtquelle detektiert werden;

- eine Zentraleinheit (35), die dazu programmiert ist:

    • eine Zeitverschiebung zwischen zwei Echos in dem von dem ausgewählten akustischen Detektor detektierten Signal zu berechnen;
    • eine Zeitverschiebung zwischen den von dem ersten Fotodetektor und dem zweiten Fotodetektor detektierten Signalen zu berechnen;
    • einen arteriellen Druck ausgehend von den berechneten Zeitverschiebungen zu schätzen.

2. Vorrichtung nach Anspruch 1, wobei die Zentraleinheit dazu programmiert ist:

    • einen arteriellen Durchmesser (D(t)) ausgehend von der Zeitverschiebung zwischen den Echos des von dem ausgewählten akustischen Detektor detektierten Signals zu schätzen;
    • eine Pulswellengeschwindigkeit (VOP) ausgehend von der Zeitverschiebung zwischen den von dem ersten Fotodetektor und dem zweiten Fotodetektor detektierten Signalen zu schätzen;
    • den arteriellen Druck (P(t)) in Abhängigkeit von dem geschätzten arteriellen Durchmesser und der geschätzten Pulswellengeschwindigkeit zu schätzen.

3. Vorrichtung nach einem der Ansprüche 1 oder 2, wobei die Lichtquelle ein Licht in einem Spektralband zwischen 500 nm und 1200 nm aussendet.

4. Vorrichtung nach einem der vorhergehenden Ansprüche, umfassend

    - eine erste Gruppe ($G_1$) von Lichtquellen und von Fotodetektoren;
    - eine zweite Gruppe ($G_2$) von Lichtquellen und von Fotodetektoren, die von der ersten Gruppe von Lichtquellen und von Fotodetektoren entfernt ist;
    - die optische Auswahleinheit ist dazu ausgestaltet

        • die erste Lichtquelle und den ersten Fotodetektor unter den Lichtquellen und den Fotodetektoren der ersten Gruppe auszuwählen;
        • die zweite Lichtquelle und den zweiten Fotodetektor unter den Lichtquellen und den Fotodetektoren der zweiten Gruppe auszuwählen.

5. Vorrichtung nach einem der vorhergehenden Ansprüche, wobei das akustische Auswahlkriterium ein Signal-Rausch-Verhältnis ist, wobei die akustische Auswahleinheit dazu ausgestaltet ist:

    - das Signal-Rausch-Verhältnis jedes von einem akustischen Detektor detektierten Signals zu schätzen;
    - den akustischen Detektor auszuwählen, bei dem das Signal-Rausch-Verhältnis am größten ist.

6. Vorrichtung nach einem der vorhergehenden Ansprüche, wobei das optische Auswahlkriterium ein Korrelationskriterium ist, wobei die optische Auswahleinheit dazu ausgestaltet ist:

    - eine zeitliche Korrelation zwischen den zu verschiedenen Zeitpunkten von Fotodetektoren jedes Lichtquelle/Fotodetektor-Paars detektierten Signalen zu schätzen;
    - die erste Lichtquelle und den ersten Fotodetektor sowie die zweite Lichtquelle und den zweiten Fotodetektor in Abhängigkeit von der geschätzten zeitlichen Korrelation auszuwählen.

7. Vorrichtung nach einem der Ansprüche 1 bis 6, wobei das optische Auswahlkriterium ein Amplitudenkriterium ist, wobei die optische Auswahleinheit dazu ausgestaltet ist:

    - eine Amplitude eines zeitlichen Verlaufs von zu verschiedenen Zeitpunkten von Fotodetektoren jedes Lichtquelle/Fotodetektor-Paars detektierten Signalen zu schätzen;
- die erste Lichtquelle und den ersten Fotodetektor sowie die zweite Lichtquelle und den zweiten Fotodetektor in Abhängigkeit von der Amplitude auszuwählen.

8. Vorrichtung nach einem der vorhergehenden Ansprüche, wobei das optische Auswahlkriterium ein Formkriterium ist, wobei die optische Auswahleinheit dazu ausgestaltet ist:

 - eine vorbestimmte zeitliche Form zu berücksichtigen;
 - einen zeitlichen Verlauf der zu verschiedenen Zeitpunkten von den Fotodetektoren jedes Lichtquelle/Fotodetektor-Paars detektierten Signalen zu bestimmen;
 - die erste Lichtquelle und den ersten Fotodetektor sowie die zweite Lichtquelle und den zweiten Fotodetektor in Abhängigkeit von einer Korrelation zwischen dem zeitlichen Verlauf der detektierten Signale und der vorbestimmten zeitlichen Form auszuwählen.

9. Verfahren zur Schätzung eines arteriellen Drucks mithilfe einer Vorrichtung nach einem der vorhergehenden Ansprüche, wobei das Verfahren umfasst:

 a) Anordnen des Trägers (10) auf der Haut eines Benutzers, gegenüber einer Arterie (A);
 b) Aussenden mindestens einer einfallenden akustischen Welle durch einen akustischen Sender und Erfassen von akustischen Signalen durch einen akustischen Detektor, wobei jedes detektierte akustische Signal Echos umfasst, die für Reflexionen der einfallenden akustischen Welle durch die Arterie repräsentativ sind, wobei der Schritt b) für verschiedene akustische Sender und/oder verschiedene akustische Detektoren ausgeführt wird, so dass jedes detektierte akustische Signal einem akustischen Sender und einem akustischen Detektor zugeordnet ist;
 c) mithilfe der akustischen Auswahleinheit:

 - Berücksichtigen eines akustischen Auswahlkriteriums;
 - Auswählen eines akustischen Senders und eines akustischen Detektors in Abhängigkeit von einem Vergleich zwischen jedem, bei dem Schritt b), detektierten akustischen Signal und von dem akustischen Auswahlkriterium;

 d) für jede Lichtquelle Aussenden eines einfallenden Lichts zu der Haut des Benutzers hin und Detektieren einer rückgestreuten Strahlung durch mindestens einen Fotodetektor, wobei jeder Fotodetektor ein optisches Signal erzeugt, das für die Intensität der rückgestreuten Strahlung repräsentativ ist;
 e) mithilfe der optischen Auswahleinheit:

 - Berücksichtigen eines optischen Auswahlkriteriums;
 - Auswählen von zwei Lichtquelle/Fotodetektor-Paaren, wobei jedes Paar eine Lichtquelle und einen Fotodetektor umfasst, in Abhängigkeit von einem Vergleich zwischen jedem aus jedem Fotodetektor hervorgehenden optischen Signal und von dem optischen Auswahlkriterium;

 f) Aussenden einer einfallenden akustischen Welle durch den bei c) ausgewählten akustischen Wandler und Bilden eines akustischen Signals, das für Echos nach Reflexionen der einfallenden akustischen Welle durch die Arterie repräsentativ ist;
 g) Aktivieren der Lichtquellen jedes bei e) ausgewählten Lichtquelle/Fotodetektor-Paars und Bilden, durch jeden Fotodetektor jedes Paars, eines optischen Signals, das für die Intensität der von der Arterie rückgestreuten Strahlung repräsentativ ist;
 h) in Abhängigkeit von einer Zeitverschiebung zwischen zwei Echos des akustischen Signals und von einer Zeitverschiebung zwischen den von jedem Detektor gebildeten optischen Signalen, Schätzen des arteriellen Drucks des Benutzers.

10. Verfahren nach Anspruch 9, wobei der Schritt h) umfasst:

 - h1) in Abhängigkeit von dem gebildeten akustischen Signal, Schätzen des Durchmessers der Arterie;
 - h2) in Abhängigkeit von den zu verschiedenen Zeitpunkten von jedem ausgewählten Fotodetektor gebildeten optischen Signalen, Schätzen einer Pulswellengeschwindigkeit;
 - h3) ausgehend von dem aus dem Teilschritt h1) resultierenden Durchmesser der Arterie und von der aus dem Teilschritt h2) resultierenden Pulswellengeschwindigkeit, Schätzen eines arteriellen Drucks des Benutzers.

11. Verfahren nach Anspruch 10, wobei der Teilschritt h2) ein Schätzen einer Zeitverschiebung ($\Delta t$) zwischen den von dem ersten Fotodetektor beziehungsweise dem zweiten Fotodetektor gebildeten optischen Signalen umfasst.

12. Verfahren nach einem der Ansprüche 9 bis 11, wobei die Schritte a) bis e) eine Kalibrierungsphase der Vorrichtung darstellen, wobei die Schritte f) bis h) zwischen zwei aufeinander folgenden Kalibrierungen wiederholt werden.

13. Verfahren nach einem der Ansprüche 9 bis 12, wobei die Vorrichtung eine Vorrichtung nach Anspruch 4 ist, wobei das Verfahren umfasst:

    - ein Auswählen eines ersten Lichtquelle/Fotodetektor-Paars aus der ersten Gruppe (G₁);
    - ein Auswählen eines zweiten Lichtquelle/Fotodetektor-Paars aus der zweiten Gruppe (G₂).

14. Verfahren nach einem der Ansprüche 9 bis 12, umfassend

    - ein Berücksichtigen eines Gültigkeitsbereichs des arteriellen Drucks;
    - wenn der aus dem Schritt h) resultierende arterielle Druck außerhalb des Gültigkeitsbereichs liegt, Wiederholen der Kalibrierungsphase.

15. Verfahren nach einem der Ansprüche 9 bis 14, wobei das akustische Auswahlkriterium ein maximales Signal-Rausch-Verhältnis ist, wobei das Auswählen des Senders und des akustischen Detektors in Abhängigkeit von dem akustischen Signal durchgeführt wird, das dem Paar akustischer Sender/akustischer Detektor zugeordnet ist, dessen Signal-Rausch-Verhältnis maximal ist.

16. Verfahren nach einem der Ansprüche 9 bis 15, wobei das optische Auswahlkriterium ein Kriterium der zeitlichen Korrelation, bis auf eine Zeitverschiebung, derart umfasst, dass das Auswählen jedes Quelle/Detektor-Paars umfasst:

    - ein Schätzen einer zeitlichen Korrelation zwischen den von den Fotodetektoren jedes Lichtquelle/Fotodetektor-Paars zu verschiedenen Zeitpunkten detektierten Signalen;
    - ein Bestimmen der Lichtquelle/Fotodetektor-Paare, für die das resultierende Signal des Fotodetektors die höchste zeitliche Korrelation aufweist.

17. Verfahren nach einem der Ansprüche 9 bis 16, wobei:

    - das erste Lichtquelle/Fotodetektor-Paar einen ersten Messpunkt (P₁) definiert;
    - das zweite Lichtquelle/Fotodetektor-Paar einen zweiten Messpunkt (P₂) definiert;
    - der erste Messpunkt und der zweite Messpunkt voneinander entfernt sind.

**Claims**

1. A device for estimation of a blood pressure of a user, the device being intended to be worn by the user, the device including:

    - a support (10) intended to be applied against the skin of the user;
    - a plurality of light sources (15) disposed on the support and configured to emit light (16) toward the skin of the user when they are activated;
    - a plurality of photodetectors (18) disposed on the support at a distance from each light source and configured to detect light (17) emanating from the skin of the user following activation of at least one light source, each photodetector forming with said light source a source - photodetector pair;
    - a plurality of acoustic transducers (11), each including at least:

        • an acoustic emitter configured to emit an acoustic wave through the skin; and
        • an acoustic detector configured to detect an acoustic wave reflected in the body of the user and propagating through the skin;

    - an acoustic selection unit (21) programmed:

        • to take into account an acoustic selection criterion;
        • in accordance with the acoustic selection criterion, to select an acoustic emitter and an acoustic detector from among the acoustic transducers, the selection being effected as a function of an acoustic signal detected by each acoustic detector following emission of an acoustic wave by at least one acoustic emitter;

    - an optical selection unit (22) configured:

        • to take into account an optical selection criterion;
        • in accordance with the optical selection criterion, to select a first light source - photodetector pair including a first light source and a first photodetector chosen from among the light sources and the photodetectors and a second light source - photodetector pair including a second light source and a second photodetector chosen from among the light sources and the photodetectors, the selection being effected as a function of the signals detected by the first photodetector and the second photodetector following activation of the first light source and of the second light source;

- a central unit (35) programmed to :

    • calculate a temporal offset between two echoes in the signal detected by the selected acoustic detector;
    • calculate a temporal offset between the signals detected by the first photodetector and the second photodetector
    • estimate a blood pressure from the calculated temporal offsets.

2. The device according to claim 1, wherein the central unit is programmed:

    • to estimate an arterial diameter (D(t)) from the temporal offset between the echoes in the signal detected by the selected acoustic detector;
    • to estimate a pulse wave velocity (VOP) from the temporal offset between the signals detected by the first photodetector and the second photodetector;
    • to estimate the blood pressure (P(t)) as a function of the estimated arterial diameter and the estimated pulse wave velocity.

3. The device according to any one of claims 1 or 2, in which each light source emits light in a spectral band between 500 nm and 1200 nm.

4. The device according to any one of the preceding claims, including

    - a first group (G$_1$) of light sources and of photodetectors;
    - a second group (G$_2$) of light sources and of photodetectors at a distance from the first group of light sources and of photodetectors;
    - the optical selection unit is then configured

        • to select the first light source and the first photodetector from among the light sources and the photodetectors of the first group;
        • to select the second light source and the second photodetector from among the light sources and the photodetectors of the second group.

5. The device according to any one of the preceding claims, wherein the acoustic selection criterion being a signal-to-noise ratio, the acoustic selection unit is configured:

    - to estimate a signal-to-noise ratio of each signal detected by an acoustic detector;
    - to select the acoustic detector for which the signal-to-noise ratio is the highest.

6. The device according to any one of the preceding claims, wherein the optical selection criterion being a correlation criterion, the optical selection unit is configured:

    - to estimate a temporal correlation between the signals detected at different times by photodetectors of each light source - photodetector pair;
    - to select the first light source and the first photodetector as well as the second light source and the second photodetector as a function of the estimated temporal correlation.

7. The device according to any one of the preceding claims, wherein the optical selection criterion being an amplitude criterion, the optical selection unit is configured:

    - to estimate an amplitude of a temporal evolution of signals detected at various times by photodetectors of each light source - photodetector pair;
    - to select the first light source and the first photodetector as well as the second light source and the second photodetector as a function of the amplitude.

8. The device according to any one of the preceding claims, wherein the optical selection criterion being a form criterion, the optical selection unit is configured:

    - to take into account a predetermined temporal form;
    - to determine a temporal evolution of the signals detected at different times by the photodetectors of each light source - photodetector pair;
    - to select the first light source and the first photodetector as well as the second light source and the second photodetector as a function of a correlation between the temporal evolution of the signals detected and the predetermined temporal form.

9. A method of estimation of a blood pressure using a device according to any one of the preceding claims, the method including:

    a) disposing the support (10) on the skin of a user, facing an artery (A);
    b) emitting at least one incident acoustic wave by means of an acoustic emitter and acquiring acoustic signals by means of an acoustic detector, each acoustic signal detected including echoes representative of reflections of the incident acoustic wave by the artery, the step b) being carried out for different acoustic emitters and/or different acoustic detectors so that each acoustic signal detected is associated with an acoustic emitter and an acoustic detector;

c) using the acoustic selection unit:

- taking into account an acoustic selection criterion;
- selecting an acoustic emitter and an acoustic detector as a function of a confrontation between each acoustic signal detected during the step b) and the acoustic selection criterion;

d) for each light source, emitting incident light toward the skin of a user and detecting back-scattered radiation by means of at least one photodetector, each photodetector generating an optical signal representative of the intensity of the back-scattered radiation;

e) using the optical selection unit:

- taking into account an optical selection criterion;
- selecting two light source - photodetector pairs, each pair including a light source and a photodetector, as a function of a confrontation between each optical signal from each photodetector and the optical selection criterion;

f) emitting an incident acoustic wave from the acoustic transducer selected in c) and forming an acoustic signal representative of echoes following reflection of the incident acoustic wave by the artery;

g) activating light sources of each light source - photodetector pair selected in e) and each photodetector of each pair forming an optical signal representative of the intensity of the radiation back-scattered by the artery;

h) estimating the blood pressure of the user as a function of the a temporal offset between two echoes in the acoustic signal and from a temporal offset between the optical signals formed by each photodetector.

10. The method according to claim 9, in which the step h) includes:

- h1) estimating the diameter of the artery as a function of the formed acoustic signal;
- h2) estimating a pulse wave velocity as a function of the optical signals formed at different times by each selective photodetector;
- h3) estimating a blood pressure of the user from the resulting diameter of the artery from the sub-step h1) and from the resulting pulse wave velocity from the substep h2).

11. The method according to claim 10, wherein the sub-step h2) includes estimating a temporal offset ($\Delta t$)

between the optical signals respectively formed by the first photodetector and the second photodetector.

12. The method according to any one of claims 9 to 11, wherein the steps a) to e) constitute a phase of calibration of the device, the steps f) to h) being reiterated between two successive calibrations.

13. The method according to any one of claims 9 to 12, wherein the device is a device according to claim 4, the method including:

- selecting a first light source - photodetector pair in the first group ($G_1$);
- selecting a second light source - photodetector pair in the second group ($G_2$).

14. The method according to any one of claims 9 to 12, including:

- taking into account a range of validity of the blood pressure;
- if the resulting blood pressure from the step h) is situated outside the range of validity, repeating the calibration phase.

15. The method according to any one of claims 9 to 14, wherein the acoustic selection criterion is a maximum signal-to-noise ratio, the selection of the acoustic emitter and of the acoustic detector being effected as a function of the acoustic signal associated with the acoustic emitter - acoustic detector pair the signal-to-noise ratio of which is the maximum.

16. The method according to any one of claims 9 to 15, wherein the optical selection criterion includes a temporal correlation criterion ignoring a temporal offset so that the selection of each source - detector pair includes:

- estimating a temporal correlation between the signals detected at different times by the photodetectors of each light source - photodetector pair;
- determining the light source - photodetector pairs for which the resulting signal from the photodetector has the highest temporal correlation.

17. The method according to any one of claims 9 to 16, wherein:

- the first light source - photodetector pair defines a first measurement point (P1);
- the second light source - photodetector pair defines a second measurement point ($P_2$);
- the first measurement point and the second measurement point are at a distance from one another.

**Fig. 1**

**Fig. 2A**

**Fig. 2B**

**Fig. 2C**

**Fig. 2D**

**Fig. 2E**

**Fig. 3A**

**Fig. 3B**

**Fig. 4**

**Fig. 5**

## RÉFÉRENCES CITÉES DANS LA DESCRIPTION

**Documents brevets cités dans la description**

- US 2010081912 A

- US 2018078155 A

**Littérature non-brevet citée dans la description**

- **NABEEL M.** Bi-modal arterial compliance probe for calibration-free cuffless blood pressure estimation. *IEEE transactions on biomedical engineering*, November 2018, vol. 5 (11)